(19) **Europäisches Patentamt / European Patent Office / Office européen des brevets**

(11) **EP 1 711 227 B1**

(12) **EUROPÄISCHE PATENTSCHRIFT**

(45) Veröffentlichungstag und Bekanntmachung des Hinweises auf die Patenterteilung:
**14.01.2009 Patentblatt 2009/03**

(21) Anmeldenummer: **05707210.0**

(22) Anmeldetag: **04.02.2005**

(51) Int Cl.:
**A61N 2/02** (2006.01)

(86) Internationale Anmeldenummer:
**PCT/EP2005/001161**

(87) Internationale Veröffentlichungsnummer:
**WO 2005/075019 (18.08.2005 Gazette 2005/33)**

(54) **VORRICHTUNG ZUR BEHANDLUNG MIT MAGNETFELDERN**

DEVICE FOR TREATING USING MAGNETIC FIELDS

DISPOSITIF DE TRAITEMENT AU MOYEN DE CHAMPS MAGNETIQUES

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **06.02.2004 DE 102004006192**

(43) Veröffentlichungstag der Anmeldung:
**18.10.2006 Patentblatt 2006/42**

(73) Patentinhaber: **Muntermann, Axel**
**35580 Wetzlar-Nauborn (DE)**

(72) Erfinder: **Muntermann, Axel**
**35580 Wetzlar-Nauborn (DE)**

(74) Vertreter: **Herden, Andreas F.**
**Blumbach - Zinngrebe**
**Patentanwälte**
**Alexandrastrasse 5**
**65187 Wiesbaden (DE)**

(56) Entgegenhaltungen:
**GB-A- 429 044        US-A- 5 143 588**
**US-A- 5 669 868        US-B1- 6 461 289**
**US-B1- 6 558 311**

**Beschreibung**

Gebiet der Erfindung

Die Erfindung betrifft eine Vorrichtung zur therapeutischen Behandlung eines Patienten mit magnetischen Feldern im Allgemeinen und eine Vorrichtung zur Behandlung mit Kernspinresonanzen im Besonderen.

Hintergrund der Erfindung

Nicht-invasive Behandlungsverfahren erobern immer neue Anwendungsgebiete in der Medizin. In Bezug auf die hier vorliegende Erfindung sind Vorrichtungen und Verfahren zur therapeutischen Behandlung mittels externer magnetischer Felder besonders hervorzuheben. Obwohl bisher die genaue Wirkungsweise solcher Therapien nicht bis ins Detail verstanden wurde, sind ihre therapeutischen Erfolge wissenschaftlich bewiesen und allgemein anerkannt. Untersuchungen zu Ergebnissen bekannter Magnetfeldtherapien finden sich z.B. in "Orthopädische Praxis" 8/2000, 36. Jahrgang, Seiten 510 bis 515 und in Fritz Lechner, "Elektrostimulation und Magnetfeldtherapie. Anwendung, Ergebnisse und Qualitätssicherung" 1989.

Insbesondere wurde in solchen Untersuchungen festgestellt, dass Magnetfeldtherapien bei den Patienten zum Teil deutliche Verbesserungen im Beschwerdebild bewirken ohne im wesentlichen nachweisbare negative Nebenwirkungen zu erzeugen. Ein weiterer großer Vorteil von Magnetfeldtherapien ist es, dass eine für den Patienten mit erheblichen Schmerzen, Risiken und Kosten verbundene Operation möglicherweise vollständig vermieden werden kann.

So ist z.B. aus der DE 40 26 173 eine Vorrichtung bekannt, welche gepulste und modulierte Magnetfelder erzeugt, um Patienten zu behandeln. Dabei wird Körpergewebe einem Magnetfeld ausgesetzt, welches sich als Überlagerung eines konstanten Magnetfeldes und eines magnetischen Wechselfeldes ergibt.

Die dort eingesetzten pulsierenden Felder benötigen jedoch viel Energie und sind träge, da die Spuleninduktivität die Feldänderung verlangsamt.

Die Therapiewirkung dieser Magnetfeldtherapie besteht unter anderem in der Linderung von Osteoporose oder den Folgen eines Schlaganfalls. Dabei erschien es wahrscheinlich, dass durch die angewandten Magnetfelder, Transport- und/oder Stoffwechselprozesse gefördert werden, die zu einer positiven therapeutischen Wirkung führen. Bislang ging man davon aus, dass die positive therapeutische Wirkung durch einen Energieaustausch zwischen Feldern und Bestandteilen von Zellen (Protonen, Ionen etc.) verursacht wird. Hierbei wurde die Energieübertragung durch die Anregung bzw. die Absorption von Ionen-Zyklotron-Resonanzen (ICR) in einem biologischen Körper erklärt und daher nach entsprechenden ICR-Bedingungen gesucht. Konsequenter Weise sind die bekannten Vorrichtungen auf die Erzeugung von ICR-Bedingungen ausgerichtet.

Allerdings erscheinen diese Ursachenerklärung und damit auch die entsprechend ausgelegten Vorrichtungen unter Umständen fraglich, da Zyklotronresonanzen im allgemeinen nur an freien Teilchen auftreten, wie beispielsweise im Vakuum oder bei Elektronen im Leitungsband eines Halbleiters. Ferner kann auch durch einfache Rechnung gezeigt werden, dass sich eine Zyklotron-Bewegung auf einer Kreisbahn vollziehen würde, deren Radius bereits den durchschnittlichen Durchmesser eines Querschnitts eines menschlichen Körpers übersteigt. Dies bedeutet, dass für den Energieübertrag eine Erklärung hinsichtlich einer Zyklotronresonanz insbesondere bei festem Gewebe fraglich sein kann.

Es ist auch denkbar, dass die Wirkung auf piezoelektrischen Vorgängen im Körper beruht. Dieser Erklärungsansatz geht davon aus, dass um jedes Körpergelenk herum ein elektrisches Feld besteht und im gesunden Zustand jede Bewegung eine Piezospannung verursacht, da der Knorpel piezoelektrische Eigenschaften besitzt. Im kranken Zustand könnten durch induzierte Spannungen diese Piezospannungen simuliert werden. Siehe hierzu auch Christian Thuile, "Das große Buch der Magnetfeldtherapie", Linz 1997.

Eine weitere Vorrichtung zur Behandlung eines biologischen Körpers mit magnetischen Feldern, welche Spinresonanzen innerhalb des zu behandelnden Körpers erzeugt, ist aus der Offenlegungsschrift WO 99/66986 bekannt. Die dort beschriebene Vorrichtung ist allerdings im wesentlichen darauf ausgerichtet eine gezielte reproduzierbare Behandlung mit Magnetfeldern in allen biologischen Materialien vorzunehmen, unabhängig davon, ob ionische Teile vorhanden sind. Mit der genannten Vorrichtung werden die positiven therapeutischen Wirkungen durch die Erzeugung von Spinresonanzen und Spinresonanzsequenzen erzielt. Hierbei wird die Kernspinresonanz aber insbesondere auch zur Energieübertragung eingesetzt.

Auf anderen Gebieten der Technik sind Kernspinresonanzverfahren (sogenannte NMR-Verfahren) bereits seit langem bekannt. Sie werden insbesondere in der medizinischen Diagnostik und allgemein für die hochpräzise Magnetfeldmessung verwendet. Zu letzterer Anwendung sei beispielhaft das "Virginia Scientific FW101 Flowing Water NMR Teslameter" genannt. Eine Beschreibung dieses Gerätes findet sich unter www.gmw.com/magnetic_measurements/VSI/FW101.html

Es bleibt festzuhalten, dass die bekannten Vorrichtungen der therapeutischen Medizin zumeist große Spulensysteme umfassen, mit denen die Magnetfelder generiert und geändert werden. Diese Spulensysteme weisen aber eine hohe Induktivität auf, was zu großen Schaltzeitkonstanten und hohem Energieverbrauch führt. Große Schaltzeiten führen aber nachteiligerweise zu einer geringen Effizienz bezüglich dynamischer Vorgänge im Körper.

Ferner sind die Spulensysteme typischerweise derart ausgebildet, dass sie Öffnungen aufweisen, in welche Körperteile, z.B. Arme oder Beine eingeführt werden können. Hierdurch sind die bekannten Vorrichtungen insgesamt relativ unförmig und weisen Nachteile bezüglich deren Lagerungs- und Transportmöglichkeiten auf. Im übrigen sind sie teilweise unbequem für den Patienten. Weiter ist der Energiebedarf der meisten bekannten Vorrichtungen sehr hoch, da mittels der Spulensysteme starke Magnetfelder erzeugt werden.

Weiter werden orthogonale Felder bei diesen bekannten Vorrichtungen mit orthogonalen Spulen erzeugt, d.h. eine horizontale zylindrische Spule erzeugt ein horizontales Magnetfeld und eine vertikale Sattel-Spule das senkrechte Feld. Das hat abermals zur Folge, dass die Vorrichtung unverhältnismäßig groß sind und nur in großen Medizin-Zentren installiert werden können.

Darüber hinaus verbleibt noch immer eine Reihe offener Fragen hinsichtlich der physikalisch-physiologischen Wirkungsweise der Vorrichtungen und der von ihnen im Körper ausgelösten Prozesse. Ohne die genaue Kenntnis der Wirkungsweise war es allerdings in der Vergangenheit nur schwer möglich einen optimierten Aufbau und die optimalen Parameter für dessen Betrieb zu bestimmen.

Seit kurzem werden für therapeutische KernspinresonanzVerfahren auch Vorrichtungen in der Fläche verwendet. Eine derartige Vorrichtung ist z.B. aus der WO 02/096514 bekannt, welche hiermit durch Referenz vollumfänglich, insbesondere in Bezug auf die physikalischen Grundlagen und medizinischen Wirkmechanismen zum Gegenstand dieser Offenbarung gemacht wird.

Insbesondere mit der letztgenannten Vorrichtung konnten bereits einige der vorstehend bezeichneten Nachteile ausgeräumt und beachtliche Behandlungserfolge erzielt werden. Dennoch hat sich gezeigt, dass die Vorrichtung z.B. in Bezug auf Ihre Größe weiter verbesserungsfähig ist.

Ferner ist die Vorrichtung nicht gleichermaßen gut zur Behandlung aller Krankheitsbilder geeignet.

Weiter kann die Vorrichtung noch besser auf die anatomischen Gegebenheiten abgestimmt werden.

Eine weitere Vorrichtung zur Behandlung mit elektromagnetischen Feldern ist aus der GB 429044 bekannt. Diese Vorrichtung bildet den nächsten Stand der Technik. Sie umfasst einen Stuhl mit Lehne zur Positionierung des Patienten und zwei an Auslegern befestigte Einrichtungen zur Erzeugung magnetischer Behandlungsfelder. Die Ausleger sind vertikal verschiebbar an seitlich neben der Lehne befindlichen Stangen befestigt. Die Anordnung der Stangen kann das Einsteigen in den Stuhl erschweren.

Allgemeine Beschreibung der Erfindung

Die Erfindung hat sich daher die Aufgabe gestellt, eine verbesserte Vorrichtung zur Behandlung eines Patienten mit magnetischen Feldern bereit zu stellen, mit welcher insbesondere eine lokalisierte Behandlung, z.B. im Kopfbereich des Patienten ermöglicht ist und welche den Patienten möglichst wenig belastet.

Eine weitere Aufgabe der Erfindung ist es, eine derartige Vorrichtung bereit zu stellen, welche platzsparend eingesetzt werden kann.

Noch eine Aufgabe der Erfindung ist es, eine derartige Vorrichtung bereit zu stellen, welche flexibel an die Anatomie des Patienten und/oder das Krankheitsbild angepasst werden kann und gute Behandlungsergebnisse liefert.

Noch eine weitere Aufgabe der Erfindung ist es, eine derartige Vorrichtung bereit zu stellen, welche kostengünstig in Herstellung und Betrieb sowie für den Patienten ansprechend und vertrauenserweckend ist.

Die Aufgabe der Erfindung wird in überraschend einfacher Weise bereits durch den Gegenstand des unabhängigen Anspruchs gelöst. Vorteilhafte Weiterbildungen der Erfindung sind in den Unteransprüchen definiert.

Erfindungsgemäß wird eine Vorrichtung zur therapeutischen Behandlung eines Patienten, insbesondere eines lebenden Menschen oder Tieres, mit Magnetfeldern vorgeschlagen, welche eine erste und eine zweite Einrichtung zum Erzeugen erster und zweiter magnetischer Behandlungsfelder umfasst. Die Vorrichtung definiert Behandlungsbereiche im welchen eine zu behandelnde Körperregion des Patienten positioniert ist, wenn der Patient auf der Vorrichtung Platz genommen hat. Anschließend wird die Behandlung mittels der Magnetfelder durchgeführt, welche in die Behandlungsbereiche eingestrahlt werden.

Die Vorrichtung umfasst ferner eine Lehne zum Anlegen des Patienten, insbesondere des Rumpfes oder Rückens des Patienten und Ausleger, welche seitlich aus der durch die Lehne definierte Ebene oder Lehnenebene herausragen, also entlang der Seite des Patienten quer zu dessen Körperachse verlaufen, wobei die Einrichtungen zum Erzeugen der magnetischen Behandlungsfelder an den Auslegern angeordnet sind und sich in einer Betriebsstellung außerhalb der Lehnenebene an der Seite des Patienten befinden. Dadurch konnen relativ kleine und lokalisierte Behandlungsfelder geschaffen werden.

Zwar geht man heute davon aus, dass derartige Magnetfeldbehandlungen keine oder nahezu keine Nebenwirkungen aufweisen, jedoch ist es dennoch von Vorteil den Behandlungsbereich relativ genau zu definieren. Hierin liegt einer der erheblichen Vorteile der Erfindung begründet. Dadurch dass die Einrichtung zur Erzeugung des ersten Magnetfeldes fast unmittelbar - ggf. lediglich durch eine textile Hülle getrennt - an die Körperoberfläche des Patienten heran geführt werden kann, können gezielt bestimmte Körperbereiche behandelt werden. Somit wird das Behandlungsfeld und damit die wenn auch nur potenzielle Belastung des Patienten auf ein Minimum reduziert.

Aufgrund der Nähe zum Patienten ergibt sich sogar ein Multiplikationseffekt. Da kleinere Spulensysteme einsetzbar sind als bei bekannten Vorrichtungen, ist die Feldkrümmung größer, d.h. das Magnetfeld hat insgesamt eine geringere räumliche Ausdehnung, so dass die Lokalisierung der Behandlung nochmals verbessert wird.

Vorzugsweise ist die Vorrichtung in Form eines Behandlungsstuhles oder -sessels ausgebildet, wobei die Lehne durch die Rückenlehne des Stuhles oder Sessels gebildet ist und die Ausleger im Bereich des Patientenkopfes angeordnet sind. Als besonders bequem für den Patienten hat sich der Einsatz eines verstellbaren Liegesessels, ähnlich einem Fernsehkomfortsessels erwiesen. Eine derartige Behandlungsvorrichtung ist besonders gut geeignet um Behandlungen im Kopfbereich des Patienten durchzuführen.

Diese Ausbildung hat den Vorteil, dass die Vorrichtung auch in kleineren Arztpraxen eingesetzt werden kann. Es liegt jedoch, z.B. zum Einsatz in größeren Behandlungszentren auch im Rahmen der Erfindung wenn die Lehne ein Abschnitt einer Behandlungsliege ist.

Nicht unterschätzt werden sollte jedoch die psychologisch vorteilhafte Wirkung eines Stuhles oder Sessels auf den Patienten im Vergleich zu einer Liege.

Es wurde im Rahmen der Erfindung herausgefunden, dass die Behandlung bei Tinnitus-Patienten eine merkliche Verbesserung des Krankheitsbildes bewirken kann. Dies ist um so überraschender, als die Ursache für Tinnitus noch wenig bekannt ist und bislang hauptsächlich medikamentöse Behandlungsversuche unternommen wurden. Mit der erfindungsgemäßen Vorrichtung kann nun erstmals gezielt im Kopf- und Halswirbelbereich behandelt werden, wobei die Bereiche in denen die Magnetfeldbehandlung wirksam ist sogar - ohne Beschränkung der Allgemeinheit - auf Teilbereiche des Kopfes, wie z.B. das Innenohr beschränkt werden kann. Es wurde nämlich herausgefunden, dass häufig Wirbelsäulenschäden oder Durchblutungsstörungen eine Ursache sein können, welche mit der erfindungsgemäßen Vorrichtung positiv beeinflussbar sind.

Die selektive Behandlung bestimmter Teilbereiche des Gehirns hat im übrigen auch bei Schlaganfällen zu positiven Ergebnissen geführt.

Weitere Behandlungserfolge können im Bereich der Kieferarthrose, Parodontose (Knochenschwäche) und bei degenerativen Kieferknochenveränderungen erzielt werden, deren Behandlung bislang ebenfalls auf klassische, in diesem Fall invasive Therapien fokussiert war. Ferner wurde herausgefunden, dass das Einwachsen von Kieferimplantaten unterstützt werden kann.

Erste Ergebnisse lassen sogar auf eine verstärkte Kollagenbildung im Behandlungsbereich schließen, so dass die erfindungsgemäße Vorrichtung unter anderem zur kosmetischen Behandlung oder bei Hauterkrankungen eingesetzt werden kann.

Vorzugsweise umfassen die Ausleger von der Lehne entfernte Anlageabschnitte, an welchen die Einrichtungen zur Erzeugung der magnetischen Behandlungsfelder angeordnet sind, wobei die Anlageabschnitte beweglich aufgehäng sind, um mit den Einrichtungen bewegbar und an die zu behandelnde Körperregion des Patienten anlegbar zu sein.

Gemäß einer vorteilhaft einfachen Ausgestaltung der Erfindung weisen die Ausleger ein oder mehrere Gelenke auf, mittels welcher die Ausleger, insbesondere in einer Ebene quer zu der Lehnenebene oder transversal, schwenkbar an der Vorrichtung aufgehängt sind, um die Bewegung der ersten Einrichtung zur Erzeugung des ersten Behandlungsfeldes zu bewerkstelligen und den Anlageabschnitt an den Körper anzulegen.

Bei der Verwendung mehrerer Gelenke sind diese zu einer Gelenkkette verbunden, wodurch eine hohe Flexibilität erreicht wird, so dass die Anlageabschnitte individuell an die Körperform des Patienten angepasst ausgerichtet werden können um diese z.B. möglichst großflächig und an die gewünschte Stelle an den Patienten anzulegen. Um die Gelenkkette zu stabilisieren, hat es sich als günstig erwiesen, ein Stabilisierungsband in die Gelenkkette einzuflechten. Auch eine mechanische Vorspannung des Auslegers hin zum Patienten kann vorgesehen sein.

Gemäß einer vorteilhaften Weiterbildung der Erfindung umfassen die Ausleger eine äußere, vorzugsweise anschmiegsame textile Hülle, welche jeweils die Einrichtungen zum Erzeugen der magnetischen Behandlungsfelder, ein Haltegestell, an welchem die erste Einrichtung befestigt ist und/oder die Gelenke umhüllt. Dadurch sind diese Elemente geschützt und die Vorrichtung ist ansehnlich und komfortabel.

Die Ausleger weisen bevorzugt einen im wesentlichen flachen Querschnitt auf, z.B. mindestens doppelt so hoch wie breit und sind insbesondere mittels des Schwenkens mit ihren flachen Seiten an die zu behandelnde Körperregion des Patienten anlegbar. Die Ausleger weisen bevorzugt einen Querschnitt von 1 cm bis 20 cm mal 2 cm bis 40 cm, insbesondere 2 cm bis 10 cm mal 5 cm bis 25 cm auf.

Der zweite Ausleger ist bevorzugt identisch wie der erste Ausleger aufgebaut, jedoch in Bezug auf den Patienten spiegelsymmetrisch zu dem ersten Ausleger. Folglich sind die beiden Anlageabschnitte aufeinander zu und voneinander weg bewegbar.

Besonders bevorzugt weist die Vorrichtung noch eine dritte Einrichtung zum Erzeugen eines dritten magnetischen Behandlungsfeldes in einem dritten Behandlungsbereich auf, wobei die dritte Einrichtung insbesondere an der Lehne parallel zu dieser angeordnet ist, so dass die erste, zweite und dritte Einrichtung eine U-förmige Anordnung bilden.

In vorteilhafter Weise sind also die erste und zweite Einrichtung seitlich am Kopf des Patienten und die dritte Einrichtung am Hinterkopf oder an der Wirbelsäule des Patienten angeordnet, wenn der Patient auf der Vorrichtung Platz genommen hat und sich die Vorrichtung in der Betriebsstellung befindet.

Mit dieser Anordnung konnte insbesondere bei der Behandlung von Tinnitus eine erhebliche Besserung der Symptome erzielt werden. Dies könnte ohne Anspruch auf Richtigkeit zu erheben, darauf zurückzuführen sein, dass gleichzeitig beide Innenohrbereiche des Patienten und bestimmte Bereiche des Kopfes und/oder der Wirbelsäule einer Behandlung unterzogen werden.

Weiter ist eine Verschiebeeinrichtung vorgesehen, an welcher der erste und zweite Ausleger aufgehängt sind, um die erste und zweite Einrichtung entlang der Vorrichtung oder Körperachse des Patienten bzw. quer zu den Auslegern zu verschieben, so dass verschiedene Körperbereiche des Patienten behandelt werden können.

Die Verschiebeeinrichtung setzt sich aus zumindest einer Schiene und einem auf dieser translatorisch entlang der Lehne, und zwar im Wesentlichen vertikal wenn die Lehne in einer aufrechten Stellung ist, bewegbaren Schlitten zusammen, wobei die Schiene an der Rückseite der Lehne befestigt ist.

Der erste und zweite Ausleger sind weiter an dem Schlitten befestigt sind, so dass die erste und zweite Einrichtung entlang der Körperachse (Symmetrieachse) des Patienten verschoben werden können.

Bevorzugt ist noch eine Verriegelungseinrichtung vorgesehen, um die Verschiebung des Schlittens auf der Schiene zu arretieren.

Weiter vorzugsweise sind der erste und zweite Ausleger lösbar an der Verschiebeeinrichtung befestigt, so dass die länglich geformten Ausleger ausgewechselt werden können, zum Beispiel um andere Körperbereiche zu behandeln oder um die Vorrichtung an den Körperbau des Patienten anpassen zu können.

Gemäß einer besonders bevorzugten Ausführungsform bilden die erste, zweite und/oder dritte Einrichtung zum Erzeugen des Magnetfeldes jeweils eine Anordnung zum Erzeugen einer Kernspinresonanz, wobei im Betrieb eine Basisspule ein Basismagnetfeld erzeugt, in welchem die anzuregenden Kerne präzedieren und mittels jeweils zwei RF-Spulen ein resonantes elektro-magnetisches Wechselfeld eingestrahlt wird.

Die Magnetfeldbehandlung erfolgt also insbesondere durch die Erzeugung von Kernspinresonanzen (NMR). Die Kernspinresonanzbedingung gemäß der Gleichung $\omega = \gamma \times B0$, wobei $\omega$ die Kreisfrequenz des Wechsel- oder RF-Feldes, $\gamma$ das gyromagnetische Verhältnis der anzuregenden Atomkerne und B0 die magnetische Induktion des Basisfeldes sind, braucht für den Fachmann hier nicht näher erläutert zu werden.

Aber auch andere Behandlungsverfahren, z.B. durch statische oder wechselnde Magnetfelder sind möglich.

Zum Erzeugen der NMR sind die Einrichtungen zum Erzeugen der magnetischen Behandlungsfelder vorzugsweise als Spulenanordnungen ausgebildet und weisen jeweils zumindest einen ersten und zweiten Magnetfelderzeuger, insbesondere in Form eines ersten bzw. zweiten Spulensystem auf. Die Behandlungsfelder werden ferner jeweils eine Überlagerung der Magnetfelder des jeweils ersten und zweiten Magnetfelderzeugers gebildet, wobei sich diese beiden Magnetfelder jeweils in dem zugehörigen Behandlungsbereich im Wesentlichen senkrecht überlagern.

Die Spulen des jeweils ersten und zweiten Spulensystems sind vorzugsweise parallel und/oder in derselben Ebene angeordnet, wodurch die erste, zweite und/oder dritte Einrichtung jeweils im Wesentlichen flache Querschnitte aufweisen, so dass eine flache Bauform erzielt werden kann. Die Ebene oder Spulenebene der ersten und zweiten Einrichtung erstreckt sich in der Betriebsstellung quer zu der Lehnenebene und/oder quer zur Körperachse des Patienten, so dass gezielt Seitenbereiche des Patienten behandelt werden können, wenn die Anlageabschnitte seitlich z.B. an den Kopf des Patienten angelegt sind. Die Spulenebene der dritten Einrichtung verläuft hingegen im Wesentlichen parallel zur Lehnenebene.

Die ersten Spulensysteme umfassen oder bestehen aus jeweils einer Basisspule und die zweiten Spulensysteme jeweils zwei RF-Spulen, insbesondere zur Erzeugung des resonanten elektro-magnetischen Wechselfeldes für die NMR.

Ferner sind jeweils die beiden RF-Spulen nebeneinander, vorzugsweise innerhalb der Öffnung der flachen Basisspule angeordnet und gegengerichtet geschaltet, so dass sich, trotz der parallelen Anordnung, zentral über der jeweiligen Einrichtung die Magnetfelder des ersten und zweiten Spulensystems senkrecht überlagern. Der jeweilige Behandlungs- oder NMR-Bereich befindet sich in einem Abstand von etwa 1 cm bis 30 cm, bevorzugt etwa 2 cm bis 10 cm, besonders bevorzugt etwa 3 cm $\pm$ 1 cm zur Oberseite der Spulen. Diese Dimensionierung hat sich für die zu behandelnden Krankheitsbilder als besonders vorteilhaft erwiesen.

Vorzugsweise beträgt die magnetische Induktion des Basismagnetfeldes für die NMR zwischen 0,1 Gauss und 1000 Gauss, insbesondere zwischen 1 Gauss und 100 Gauss. Demnach beträgt die Frequenz f des RF-Feldes für Wasserstoff entsprechend 422,5 Hz bis 4,225 MHz, vorzugsweise 4,225 kHz bis 422,5 kHz gemäß der Gleichung

$$f \; [kHz] \; = \; 4{,}225 \; x \; B0[Gauss].$$

Besonders bevorzugt wird die Kernspinresonanz periodisch erzeugt, wobei die Wiederholfrequenz der periodischen Kernspinresonanzanregung vorzugsweise 1 Hz bis 1000 Hz, insbesondere 5 Hz bis 100 Hz, besonders bevorzugt bis 40 Hz beträgt, wobei insbesondere das RF-Feld mit dieser Periode diskontinuierlich, z.B. innerhalb einer Rechteck-Umhüllenden eingestrahlt wird.

Im Folgenden wird die Erfindung anhand von Ausführungsbeispielen und unter Bezugnahme auf die Zeichnungen näher erläutert, wobei gleiche und ähnliche Elemente teilweise mit gleichen Bezugszeichen versehen sind und Merkmale verschiedener Ausführungsbeispiele miteinander kombinierbar sind.

Kurzbeschreibung der Figuren

Es zeigen:

Fig. 1      eine schematische Seitenansicht eines erfindungsgemäßen Behandlungsstuhles,
Fig. 2      eine schematische Draufsicht von oben auf den Behandlungsstuhl aus Fig. 1,
Fig. 3      eine schematische Rückansicht einer Verschiebeeinrichtung an dem Behandlungsstuhl,
Fig. 4      eine schematische Draufsicht von oben auf die Verschiebeeinrichtung aus Fig. 3 mit Auslegern,
Fig. 5      eine ausschnittsweise Seitenansicht eines Auslegers mit Gelenkkette,
Fig. 6      einen schematischen Querschnitt entlang der Linie 6-6 in Fig. 7 durch eine erfindungsgemäße Einrichtung zur Erzeugung eines Behandlungsfeldes,
Fig. 7      einen schematischen Querschnitt durch die Einrichtung aus Fig. 6 entlang der Linie 7-7,
Fig. 8      ein Foto der Einrichtung aus Fig. 7,
Fig. 9      ein Foto einer Einrichtung zur Erzeugung des Behandlungsfeldes gemäß einer weiteren Ausführungsform der Erfindung,
Fig. 10     ein Blockschaltbild eines Steuergeräts,
Fig. 11     eine beispielhafte Darstellung des zeitlichen Verlaufs der Intensität der Magnetfelder und

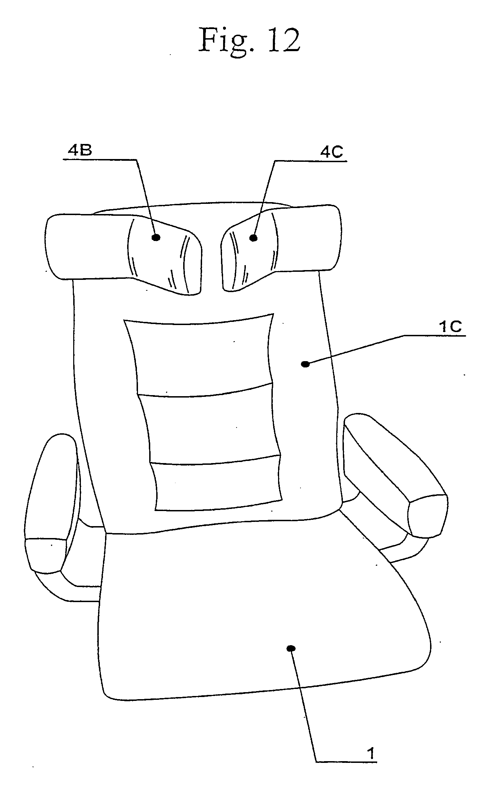

Fig. 12     ein Foto einer Ausführungsform des erfindungsgemäßen Behandlungsstuhls.

Detaillierte Beschreibung der Erfindung

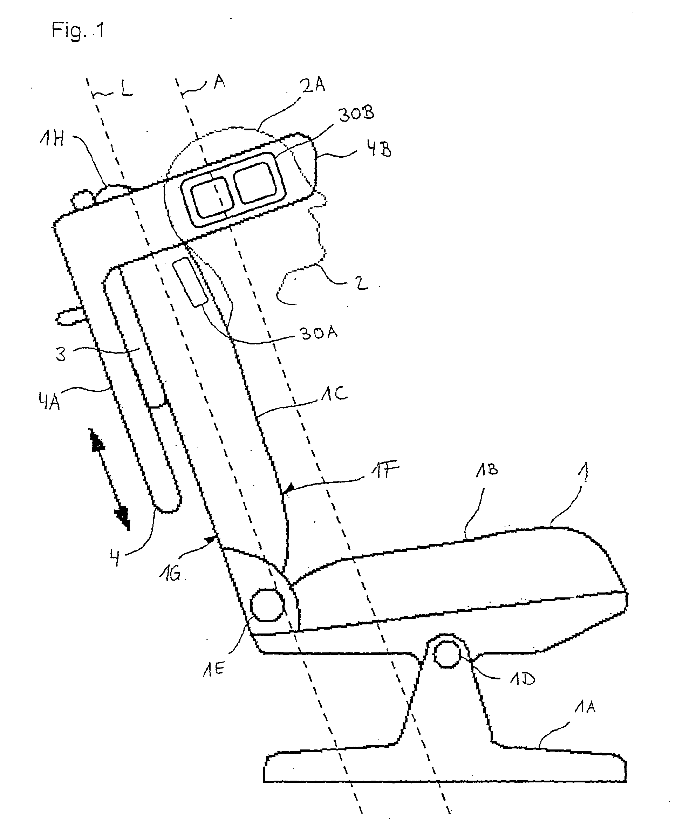

Bezug nehmend auf Fig. 1 ist die erfindungsgemäße Vorrichtung zur therapeutischen Behandlung mit magnetischen Feldern in Form eines Behandlungsstuhles 1 dargestellt. Der Behandlungsstuhl 1 umfasst einen Fuß 1A, eine Sitzfläche 1B und eine Lehne oder Rückenlehne 1C, welche eine im Wesentlichen parallel zur Körperachse A verlaufende Lehnenebene L definiert. Der Behandlungsstuhl 1 ist ähnlich einem Relax-Sessel derart ausgebildet, dass die Sitzfläche 1B um ein Gelenk 1D und die Rückenlehne 1C um ein Gelenk 1E neigbar sind, um für den Patienten eine komfortable Behandlungsposition bereitzustellen.

Zur Behandlung setzt sich der Patient 2, von dem der Übersicht halber lediglich der Kopf 2A dargestellt ist, auf den Behandlungsstuhl 1 und lehnt mit seinem Rücken an der Vorderseite 1F der Rückenlehne 1C.

An einer der Vorderseite 1F gegenüberliegenden Rückseite 1G der Rückenlehne 1C ist eine Verschiebeeinrichtung 3 befestigt, an welcher ein Haltegestell 4 verschiebbar entlang der Rückenlehne 1C oder parallel zur Körperachse A des Patienten 2 aufgehängt ist.

Das Haltegestell umfasst einen Halteabschnitt 4A und zwei spiegelsymmetrische Ausleger 4B, 4C, von denen in Fig. 1 lediglich der rechte Ausleger 4B zu sehen ist und wobei der Halteabschnitt 4A und die Ausleger 4B, 4C in der Seitenansicht im Wesentlichen eine L-Form oder einen rechten Winkel bilden. Die im Wesentlichen schmalen seitlichen Ausleger 4B, 4C erstrecken sich scheuklappenartig von hinten nach vorne oder quer zur Körperachse A entlang des Patientenkopfes 2A.

An den seitlichen Auslegern 4B, 4C ist jeweils eine Einrichtung 30B, 30C zur Erzeugung des Behandlungsfeldes in Form von jeweils einer Spulenanordnung 30B, 30C befestigt. Hierzu sind die beweglichen Ausleger 4B, 4C zum Beispiel als Halterahmen aus nicht magnetischem Material ausgebildet und die Spulenanordnungen 30B, 30C sind innerhalb des jeweiligen Rahmens befestigt.

Innerhalb der Rückenlehne 1C ist eine dritte Spulenanordnung 30C etwa im Hinterkopf- oder Halswirbelbereich des Patienten 2 angeordnet.

Alternativ kann die dritte Spulenanordnung 30A auch außerhalb der Rückenlehne 1C an deren Vorderseite 1F angebracht und mit einem Halteband, welches über die Oberseite 1H der Rückenlehne 1C und dem Halteabschnitt 4A verläuft, aufgehängt sein. In diesem Fall ist das eine Ende des Haltebandes mit der dritten Spulenanordnung 30A und das zweite Ende des Haltebandes mit der Rückenlehne 1C verbunden, so dass die dritte Spulenanordnung 30A automatisch mit dem Haltegestell 4 entlang der Körperachse A verschoben wird. Bei dieser Ausführungsform ist gewährleistet, dass die drei Spulenanordnungen 30A, 30B, 30C bei einer Verschiebung des Haltegestells 4 zueinander zumindest in etwa dieselbe Position behalten.

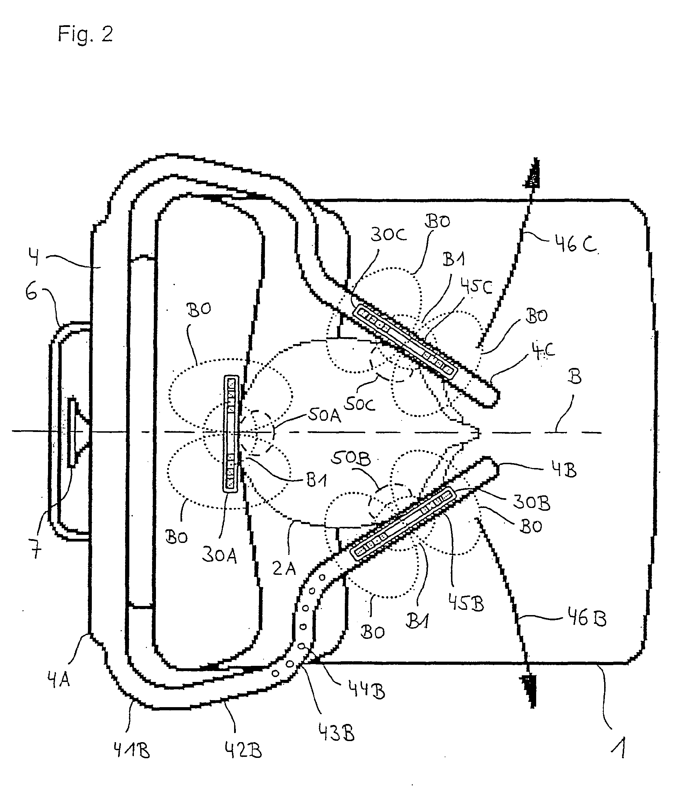

Bezug nehmend auf Fig. 2 ist der Behandlungsstuhl 1 mit dem Patienten 2 in einer Draufsicht von oben schematisch dargestellt, wobei die Spulenanordnungen 30A, 30B und 30C - obwohl in der Realität verdeckt - in der Darstellung ebenfalls gezeigt sind.

Der rechte Ausleger oder Spulenausleger 4B ist mit einem gebogenen Anschlussabschnitt 41B an dem Halteabschnitt 4A befestigt, erstreckt sich anschließend mit einem geraden Abschnitt 42B nach vorne und umfasst anschließend einen Gelenkabschnitt 43B mit einer Mehrzahl von Gelenken 44B, welche eine Gelenkkette bilden, um den Gelenkabschnitt vielfältig verformen zu können. In der dargestellten Betriebsstellung hat der Gelenkabschnitt 43B im Wesentlichen eine S-Form.

An einem in Bezug auf den Halteabschnitt 4A entfernten und sich an den Gelenkabschnitt 44B anschließenden im Wesentlichen geraden Anlageabschnitt 45B ist die Spulenanordnung 30B befestigt. Der am vorderen Ende des Auslegers 4B befindliche Anlageabschnitt 45B sowie die Spulenanordnung 30B erstrecken sich in diesem Beispiel zur Behandlung des linken und rechten Gesichtsbereiches in der Draufsicht von oben schräg nach vorne und innen und die Spulenanordnungen 30A, 30B und 30C bilden eine U-Form bzw. verlaufen entlang der Seiten eines Dreiecks. Somit sind die Spulen derart angeordnet, dass die Magnetfeldlinien jeweils quer zur Lehne und quer zur Körperachse A aus den Spulen austreten.

Mittels der Gelenke 44B lässt sich der Ausleger 4B etwa entlang des Pfeils 46B von dem Patientenkopf 2A weg- bzw. wieder auf diesen zu bewegen, genauer schwenken, derart, dass der Anlageabschnitt 45B mit der Spulenanordnung 30B bewegbar, genauer von dem Patienten weg bzw. auf den Patienten zu bewegbar ist. Durch die Kombination mit der Verschiebeeinrichtung 3 sind die Spulenanordnungen 30B, 30C also zumindest zweidimensional bewegbar.

In der Betriebsstellung liegt der Anlageabschnitt 45B an der zu behandelnden Körperregion des Patienten 2 an oder befindet sich zumindest in unmittelbarer Nähe- Der Anlageabschnitt 45B ist zusammen mit der darin angeordneten Spulenanordnung 30B von einer textilen Hülle, Kunststoffhülle oder anderen verkleidenden Materilien umgeben, welche in den Figuren nicht dargestellt ist.

Die gesamte Anordnung ist spiegelsymmetrisch um die Symmetrieachse B des Patienten ausgebildet, so dass der linke Ausleger 4C und die linke Spulenanordnung 30C identisch aber spiegelverkehrt ausgebildet sind.

Jede der drei Spulenanordnungen 30A, 30B und 30C erzeugt ein Basismagnetfeld B0 und ein RF-Feld B1, welche sich in dem jeweiligen Behandlungsbereich 50A, 50B, 50C, die sich zumindest teilweise im Inneren des Patientenkörpers befinden, im Wesentlichen senkrecht zu dem jeweiligen Behandlungsfeld überlagern. Dabei sind die Behandlungsbereiche 50A, 50B, 50C jeweils nach innen aufeinander und auf den Patienten zugewandt. Mit anderen Worten befindet sich die zu behandelnde Körperregion des Patienten innerhalb des jeweiligen Behandlungsfeldes.

Die drei Behandlungsbereiche sind naturgemäß nicht exakt abgrenzbar und daher lediglich schematisch durch gestrichelte Linien mit den Bezugszeichen 50A, 50B und 50C veranschaulicht.

Die Größe und Position der jeweiligen Behandlungsbereiche 50A, 50B und 50C lässt sich in gewissen Grenzen durch die Abstimmung der magnetischen Felder variieren. Selbstverständlich können die drei Behandlungsbereiche 50A, 50B, 50C sich auch zu einem gemeinsamen Behandlungsbereich überlappen.

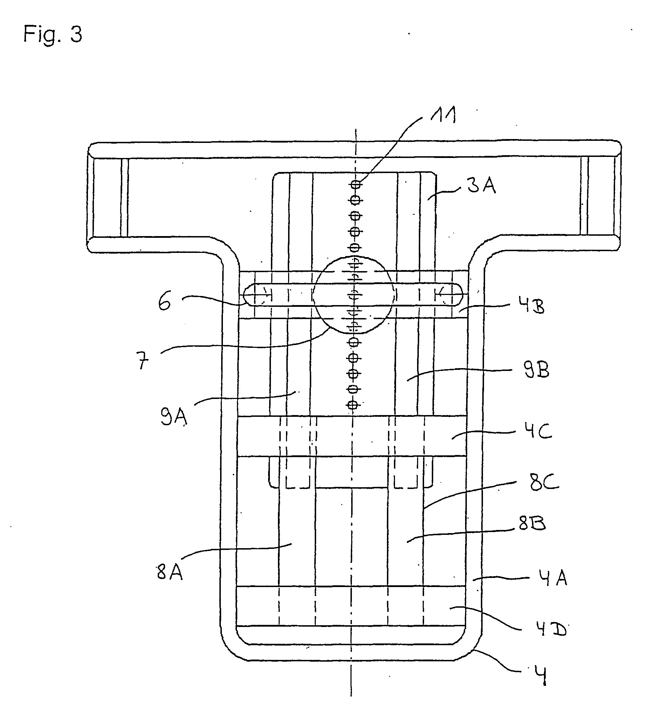

Bezug nehmend auf Fig. 3 ist eine Rückansicht des Haltegestells 4 gezeigt.

Das Haltegestell 4 umfasst den Halteabschnitt 4A, eine obere, mittlere und untere Querstrebe 4B, 4C, 4D und zwei Verstellschienen 8A, 8B, welche einen Verstellschlitten 8C bilden. Der Verstellschlitten 8C gleitet auf einer Schiene in Form einer Adapterplatte 3A, welche an der Rückseite 1G der Rückenlehne 1C befestigt ist.

Die Adapterplatte 3A weist eine Mehrzahl von vertikal angeordneten Bohrungen 11 auf, in welche ein Raststift, welcher an einem Zugknopf 7 angebracht ist, als Arretiermittel in der jeweils gewünschten Stellung einrastet, um die Verschiebung des Haltegestells 4 zu arretieren. Der Zugknopf 7 ist hierbei im Bereich des Verstellgriffs 6 angeordnet, um eine leichte Handhabbarkeit zu gewährleisten.

Für Service und Reparaturzwecke oder andere medizinische Indikationen ist das Haltegestell 4 zusammen mit den Auslegern 3B und 3C von dem Stuhl 1 abnehmbar und austauschbar. Hierzu wird eine Sicherung gelöst und der Verstellschlitten 8C entlang der Schienen nach oben abgezogen. An dem Halteabschnitt 4A ist noch ein Stellgriff 6 zum manuellen Verschieben des Haltegestells 4 fest angebracht.

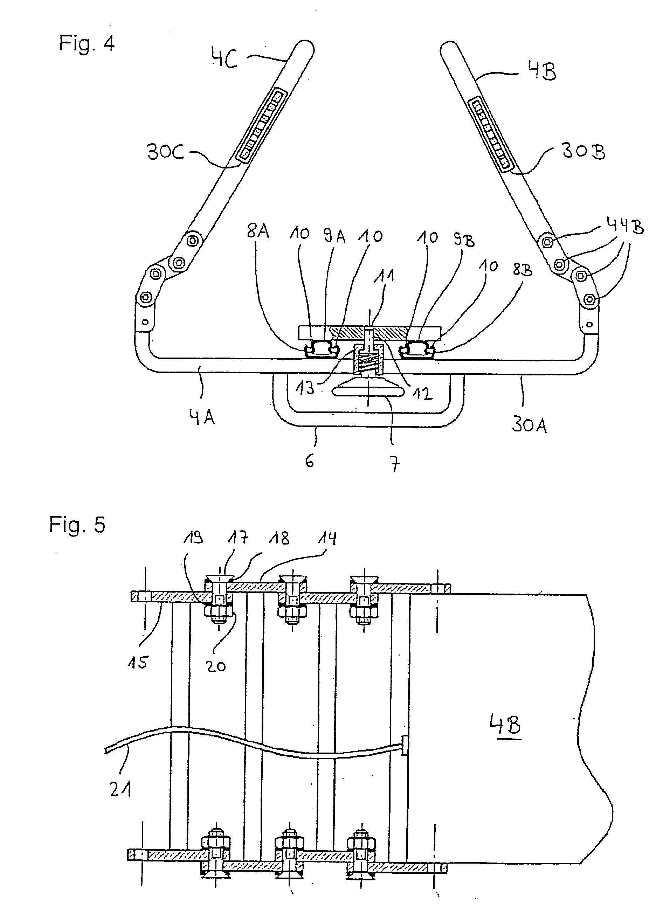

Bezug nehmend auf Fig. 4 ist eine aufgebrochene Draufsicht von oben auf den Verstellmechanismus und die Ausleger ohne die textile Umhüllung dargestellt.

In der Darstellung ist insbesondere zu sehen, wie die Verstellschienen 8A, 8B an den Verstellschienen 9A, 9B der Adapterplatte mittels Kugeln 10 kugelgelagert verschiebbar sind. Ferner ist der Eingriff des Raststiftes 12 in die Bohrung 11 des Lochrasters dargestellt.

Weiter ist die flexible transversale Verstellmöglichkeit der Ausleger 30B, 30C mittels der Gelenke 44B und dazwischen liegenden Gliederelementen 14, 15 gezeigt, welche gemeinsam die Gelenkkette jedes Auslegers bilden.

Die Ausleger 30B, 30C sind mit nicht dargestellten Befestigungsmitteln lösbar an dem gemeinsamen Halteabschnitt 4A befestigt.

Bezug nehmend auf Fig. 5 ist eine Seitenansicht der Gliederelemente 14, 15 gezeigt, welche paarweise mittels Druckelementen 17, Druckringen 18, Scheiben 19 und Muttern 20 leicht geklemmt drehbar miteinander verbunden sind. Ferner ist eine elektrische Zuleitung 21 für die Spulenanordnung dargestellt.

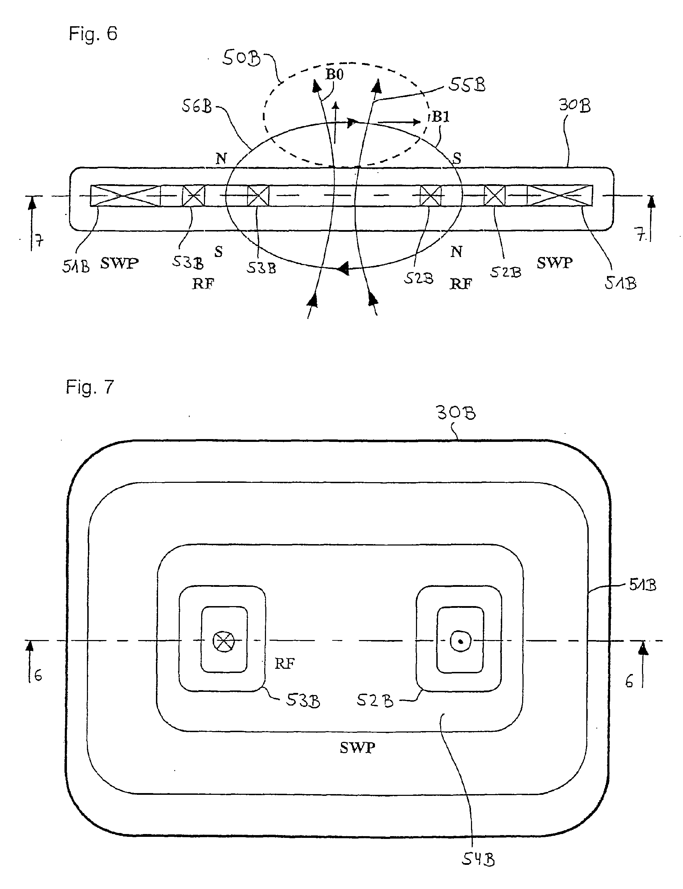

Bezug nehmend auf Fig. 6 ist, stellvertretend für alle drei identischen Spulenanordnungen 30A, 30B, 30C, die Spulenanordnung 30B schematisch in einem Querschnitt parallel zur Spulenebene dargestellt.

Die Spulenanordnung 30B umfasst eine Basisspule 51B zur Erzeugung des quasi-stationären Basisfeldes B0, dessen magnetische Feldlinien 55B zentral senkrecht aus der Spulenanordnung 30B austreten, sowie zwei RF-Spulen 52B, 53B.

Die beiden RF-Spulen 52B, 53B sind entgegengesetzt gepolt, so dass die magnetischen Feldlinien 56B des magnetischen Wechselfeldes B1 an der Oberseite der linken RF-Spule 53B nach oben austreten und an der Oberseite der rechten RF-Spule 52B wieder in die Spulenanordnung eintreten. Dadurch wird im Behandlungsbereich 50B erreicht, dass die mittels parallel angeordneter Spulen 51B, 52B, 53B erzeugten Magnetfelder B0 und B1 trotz parallelem Austritt aus den Spulen im Wesentlichen senkrecht aufeinander stehen und Kernspinresonanz-Bedingungen in dem Behandlungsbereich 50B erfüllt werden können.

Bezug nehmend auf Fig. 7 ist eine Schnittdarstellung entlang der Linie 7-7 in Fig. 6 dargestellt, wobei die Linie 7-7 die Spulenebene repräsentiert. 6-6 zeigt die Schnittlinie für die Darstellung in Fig. 6.

In Fig. 7 ist zu erkennen, dass die beiden RF-Spulen 52B, 53B innerhalb der Öffnung 54B zu der Basisspule 51B angeordnet sind.

Durch die erfindungsgemäße planare Anordnung der Basisspulen 51B und der RF-Spulen 52B und 53B, gelingt es orthogonale Magnetfelder im Behandlungsbereich zu generieren. Dabei produziert die Basis- oder Sweepspule 51B im Behandlungsbereich das in Bezug auf die Spulenebene vertikale quasistatische Magnetfeld B0 und die zwei Radiofrequenz-Spulen 52B, 53B innerhalb der Sweepspule im Behandlungsbereich das in Bezug auf die Spulenebene parallele magnetische Wechselfeld B1.

Die Form der Spulenanordnungen kann weiter der Anwendung und der Körperform entsprechend angepasst z.B. für Gelenkschäden gebogen sein. Eine derartige Ausführungsform der Erfindung (nicht dargestellt) hat sich besonders für die Behandlung von Tieren bewährt. Z.B. kann die Spulenanordnung an einer Gamasche befestigbar, insbesondere anheftbar sein, welche z.B. bei der Knöchelbehandlung von Pferden eingesetzt wird.

Es wurde auch eine weiche Behandlungsdecke mit Erfolg eingesetzt, welche über den Rücken eines Pferdes gelegt wird und an welcher eine oder mehrere Spulenanordnungen an beliebiger Stelle, z.B. mittels Klettband, lösbar befestigbar sind.

Die RF-Spulen 52B, 53B besitzen ferner eine serielle Induktivität L und bilden mit einem Kondensator C einen Schwingkreis. Für die elektrische Eigenresonanzfrequenz F gilt:

$$F = \frac{1}{2\pi\sqrt{LC}}$$

Dabei sind die Eigenresonanzfrequenz und die Kernspinresonanzfrequenz aufeinander abgestimmt, so dass der RF-Sender im Steuergerät automatisch eine Wechselspannung mit der Frequenz F generiert, um möglichst große Wechselfelder mit relativ kleiner Energie zu erzeugen.

Jede der Spulenanordnungen 30A, 30B, 30C erzeugt die für das Kernspinresonanzverfahren jeweils erforderlichen zwei orthogonalen Magnetfelder B0 und B1 gemäß der für Protonen, welche etwa 80% der im menschlichen und tierischen Körper vorkommenden Atomkerne ausmachen, gültigen Gleichung

$$f[kHz] = 4{,}225 \times B0[Gauss]$$

Das magnetische Behandlungsfeld B0 setzt sich dabei zusammen aus einer konstanten oder statischen Komponente B01 und einer kleineren Modulationskomponente B02, d.h. B0 ist quasi-statisch. Die Frequenz f wird dabei auf den statischen Anteil B01 abgestimmt.

Zum Beispiel beträgt B01 = 4 Gauss und die Frequenz f = 16,9 kHz.

Der Betrag der variablen Magnetfeldkomponente B02 beträgt etwa 10% bis 100%, vorzugsweise 20% bis 70%, am meisten bevorzugt um etwa 50% $\pm$ 10 %-Punkte des Betrages von B01. Durch die Variation der Modulationskomponente B02 wird die Inhomogenität von B01 kompensiert. Mit anderen Worten wird das Basismagnetfeld B0 derart moduliert, dass die Kernspinresonanzbedingung zumindest zeitweise über den vollständigen Behandlungsbereich erfüllt ist. Mit anderen Worten wird die natürliche Inhomogenität von B0 in Kombination mit der Modulation mittels B02 dazu eingesetzt um die Resonanzbedingung über den Behandlungsbereich zu scannen.

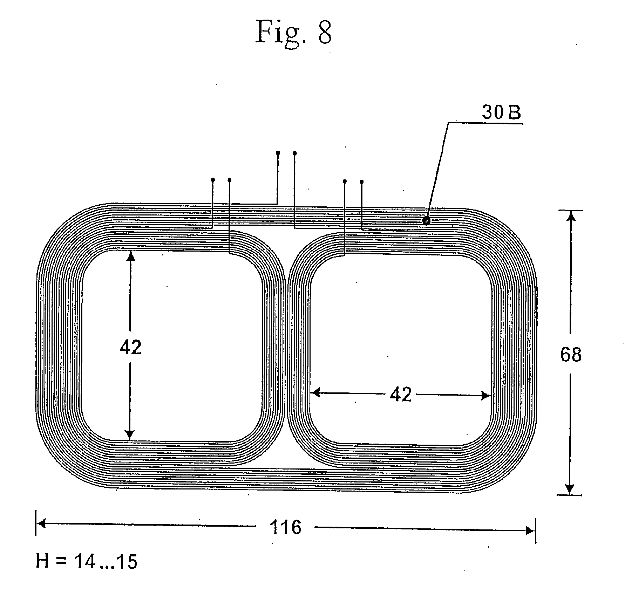

Bezug nehmend auf Fig. 8 ist ein Foto der Spulenanordnung 30B mit Bemaßung dargestellt. Die Spulenanordnung weist eine Länge L = 116 mm, eine Breite B = 68 mm und eine Höhe H = 15 mm auf. Die Öffnung der jeweils im Wesentlichen quadratischen RF-Spulen 52B, 53B beträgt etwa 42 mm im Quadrat. Eine Variation der Größe auf einen Bereich von etwa einem Zwanzigstel, Fünftel, Drittel oder einer Hälfte bis etwa mal zwei, drei, fünf oder zwanzig der angegebenen Maße liegt jedoch auch im Rahmen der Erfindung.

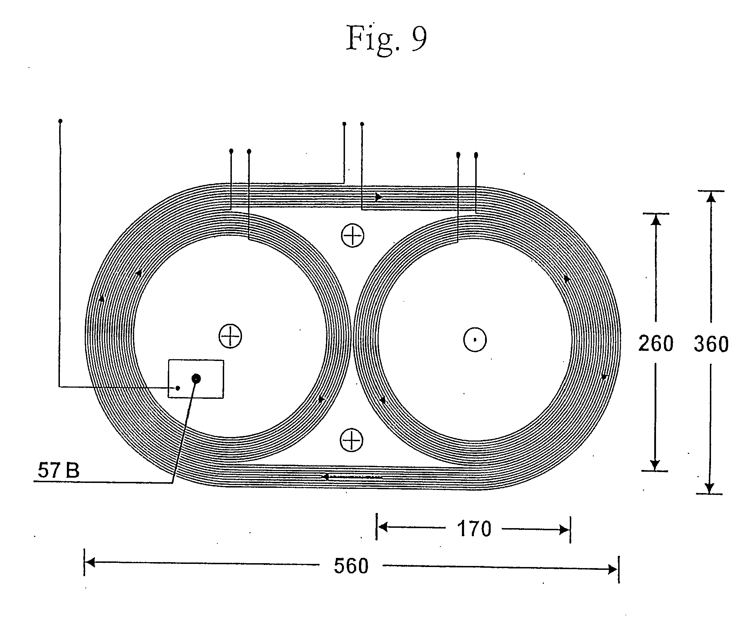

Bezug nehmend auf Fig. 9 ist eine alternative Ausführungsform des Spulensystems mit im Wesentlichen kreisförmigen RF-Spulen und einer ovalen Basisspule um die RF-Spulen dargestellt. Ferner ist ein Abstimmglied 57B vorgesehen, welches innerhalb der Spulenanordnung angebracht ist. Mittels des Abstimmgliedes 57B wird das NMR-Signal detektiert und das Basisfeld B0 und/oder das RF-Feld B1 angepasst, so dass ein Regelkreis zur Steuerung der Magnetfelder für die Kernspinresonanz gebildet ist.

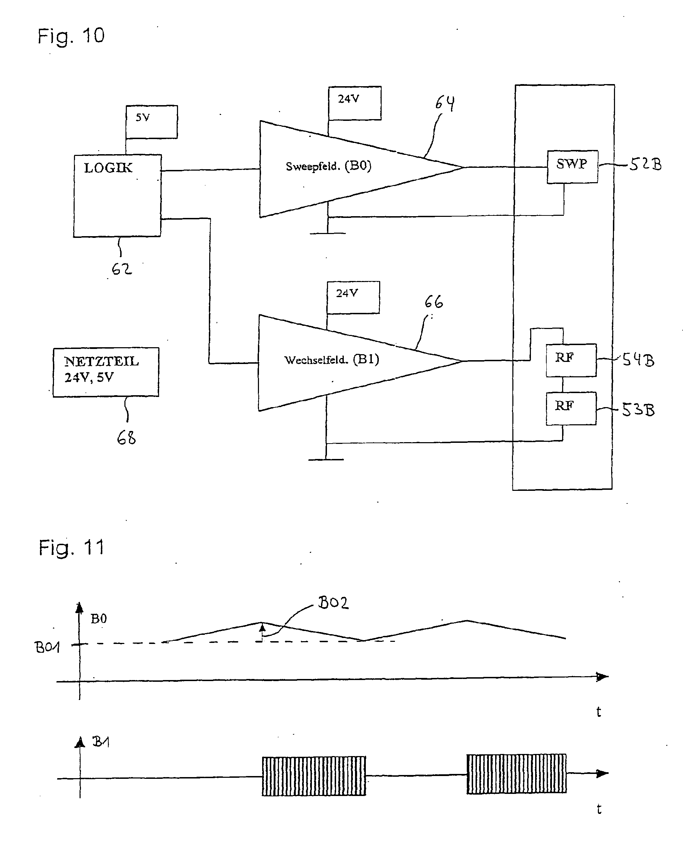

Bezug nehmend auf Fig. 10 ist ein Blockschaltbild der Steuerelektronik dargestellt. Ein Logikbaustein 62 steuert eine Ansteuereinrichtung 64 für die Basis- oder Sweep-Spule 51B und eine Ansteuereinrichtung 66 steuert die beiden RF-Spulen 52B, 53B, welche in Serie geschaltet sind. Ferner wird die Vorrichtung mittels eines Netzteils 68 mit Strom versorgt. Das Steuergerät kann dabei eine, zwei, drei oder mehr Spulenanordnungen ansteuern.

Bezug nehmend auf Fig. 11 ist ein beispielhafter Magnetfeldverlauf für eine periodische Kernspinresonanz-Erzeugung dargestellt. Das Basisfeld B0 weist einen konstanten Sockelbetrag B01 und einen zeitlich variablen Anteil B02, in diesem Beispiel entsprechend einer Dreiecksmodulation, auf. Das Wechselfeld B1 wird diskontinuierlich periodisch mit einer Rechteck-Umhüllenden während der abfallenden Flanken des Basisfeldes B0 eingestrahlt. Mit anderen Worten ist das Wechselfeld B1 an den fallenden Flanken von B0 aktiv und an den steigenden Flanken gleich null, was auch als schneller adiabatischer Durchlauf ("Fast Adiabatic Passage") bezeichnet wird. Die Wasserstoffkernmagnetisierung im Körper wird dabei jeweils um 180° gekippt.

In der nachfolgenden Tabelle 1 sind besonders vorteilhafte Werte für die Behandlung angegeben.

Tabelle 1:

| | |
|---|---|
| Wechselfeld (B1) | 2 kHz bis 40 kHz, insbesondere um etwa 30 kHz |
| B1-Amplitude | 0,02 mT bis 0,15 mT, insbesondere um etwa 0,05 mT |
| quasi-statisches Magnetfeld (B01) | 30 mm über dem Spulensystem 0,1 mT bis 1,0 mT, insbesondere um etwa 0,7 mT |
| Magnetfeldsweep (B02) | 20 % bis 50 % von B01, insbesondere um etwa 30 % |
| Modulationsfrequenz von B02 | 1 Hz bis 250 Hz |
| Modulationsart von B02 | Dreieck, Rechteck, Sinus, besonders bevorzugt Dreieck |

Bei größeren Spulenanordnungen haben sich allerdings auch Frequenzen des Wechselfeldes B1 von 5 kHz bis 1 MHz bewährt.

**Patentansprüche**

1. Vorrichtung zur therapeutischen Behandlung eines Patienten (2) mit Magnetfeldern (B0, B1, 55B, 56B) umfassend

   eine erste Einrichtung (30B) zum Erzeugen eines ersten magnetischen Behandlungsfeldes (B0, B1, 55B, 56B) innerhalb eines ersten Behandlungsbereiches (50B),
   eine zweite Einrichtung (30C) zum Erzeugen eines zweiten magnetischen Behandlungsfeldes (B0, B1, 55B, 56B) innerhalb eines zweiten Behandlungsbereiches (50C),
   eine Lehne (1C) zum Anlegen des Patienten (2), derart dass eine zu-behandelnde Körperregion des Patienten (2) in dem ersten Behandlungsbereich (50B) und dem zweiten Behandlungsbereich (50C) positioniert ist, wenn der Patient (2) auf der Vorrichtung Platz genommen hat und sich die Vorrichtung in einer Betriebsstellung befindet,

einen ersten Ausleger (4B) und einen zweiten Ausleger (4C), welche aus der durch die Lehne (1C) definierte Ebene herausragen, wobei die erste Einrichtung (30B) zum Erzeugen des ersten magnetischen Behandlungsfeldes an dem ersten Ausleger (4B) angeordnet ist, wobei die zweite Einrichtung (30C) zum Erzeugen des zweiten magnetischen Behandlungsfeldes an dem zweiten Ausleger (4C) angeordnet ist, derart, dass ein Körperbereich des Patienten (2) zwischen der ersten und zweiten Einrichtung (30B, 30C) positionierbar ist; wobei die Vorrichtung eine Verschiebeeinrichtung (3A, 4A) umfasst, an welcher der erste und zweite Ausleger (4B, 4C) aufgehängt sind, um die erste und zweite Einrichtung (30B, 30C) im Wesentlichen entlang der Körperachse (1) des Patienten (2) zu verschieben, wobei die Verschiebeeinrichtung (3A, 4A) zumindest eine Schiene (9A, 9B) umfasst, welche an der Rückseite (1G) der Lehne (1C) befestigt ist und der erste und zweite Ausleger (4B, 4C) an einem Schlitten (8A, 8B) befestigt sind, welcher an der zumindest einen Schiene (9A, 9B) entlang der Lehne (1C) verschieblich angeordnet ist.

2. Vorrichtung nach Anspruch 1,
wobei die Vorrichtung in Form eines Behandlungsstuhles (1) ausgebildet ist und die Lehne (1C) durch die Rückenlehne des Behandlungsstuhls (1) gebildet wird.

3. Vorrichtung nach einem der vorstehenden Ansprüche,
wobei die Ausleger jeweils einen von der Lehne entfernten Anlageabschnitt (45B, 45C) umfassen, an welchen die erste und zweite Einrichtung zur Erzeugung der ersten und zweiten magnetischen Behandlungsfelder angeordnet sind, und wobei die Anlageabschnitte beweglich aufgehängt und an die zu behandelnde Körperregion des Patienten anlegbar sind.

4. Vorrichtung nach einem der vorstehenden Ansprüche,
wobei die Ausleger schwenkbar an der Vorrichtung angebracht sind.

5. Vorrichtung nach seinem der vorstehenden Ansprüche,
wobei die Ausleger in einer Ebene quer zu der Lehnenebene schwenkbar ausgebildet und seitlich an den Patienten (2) anlegbar sind.

6. Vorrichtung nach einem der vorstehenden Ansprüche,
wobei die Ausleger mehrere Gelenke (44B) umfassen, welche jeweils eine Gelenkkette bilden.

7. Vorrichtung nach Anspruch 6,
wobei jeweils ein Stabilisierungsband durch die Gelenkketten geflochten ist.

8. Vorrichtung nach einem der vorstehenden Ansprüche,
wobei die Ausleger jeweils eine äußere Hülle umfassen und die erste und zweite Einrichtung zum Erzeugen der ersten und zweiten magnetischen Behandlungsfelder und die jeweiligen Gelenke jeweils innerhalb der Hülle angeordnet sind.

9. Vorrichtung nach einem der vorstehenden Ansprüche,
wobei die Ausleger einen im wesentlichen flachen Querschnitt aufweisen und mittels des Schwenkens jeweils mit ihrer flachen Seite an die zu behandelnde Körperregion des Patienten (2) anlegbar sind.

10. Vorrichtung nach einem der vorstehenden Ansprüche,
wobei die Vorrichtung eine dritte Einrichtung (30A) zum Erzeugen eines dritten magnetischen Behandlungsfeldes in einem dritten Behandlungsbereich (50A) umfasst, wobei die dritte Einrichtung (30A) an der Lehne (1C) angeordnet ist und wobei die erste, zweite und dritte Einrichtung (30B, 30C, 30A) im Wesentlichen U-förmig angeordnet sind.

11. Vorrichtung nach einem der vorstehenden Ansprüche,
wobei die erste und zweite Einrichtung (30B, 30C) seitlich am Kopf (2A) des Patienten (2) und die dritte Einrichtung (30A) im Bereich des Hinterkopfes oder der Wirbelsäule des Patienten (2) angeordnet sind, wenn der Patient auf der Vorrichtung Platz genommen hat und sich die Vorrichtung in der Betriebsstellung befindet.

12. Vorrichtung nach einem der vorstehenden Ansprüche,
wobei eine Verriegelungseinrichtung (7, 11, 12) umfasst ist; um die Verschiebung des Schlittens (8A, 8B) zu arretierten.

**13.** Vorrichtung nach einem der vorstehenden Ansprüche,
wobei der erste und zweite Ausleger (4B, 4C) lösbar an der Verschiebeeinrichtung (3A, 4A) befestigt sind, um die Ausleger (4B, 4C) auszuwechseln.

**14.** Vorrichtung nach einem der vorstehenden Ansprüche,
wobei die erste Einrichtung (30B) zum Erzeugen des ersten magnetischen Behandlungsfeldes zumindest einen ersten (51B) und zweiten (52B, 53B) Magnetfelderzeuger umfasst, wobei der erste und zweite Magnetfelderzeuger als erstes bzw. zweites Spulensystem ausgebildet sind.

**15.** Vorrichtung nach Anspruch 14,
wobei das erste magnetische Behandlungsfeld durch eine Überlagerung der Magnetfelder (B0, B1, 55B, 56B) des ersten und zweiten Magnetfelderzeugers (51B, 52B, 53B) gebildet wird, wobei sich diese beiden Magnetfelder in dem ersten Behandlungsbereich (50B) im Wesentlichen senkrecht überlagern.

**16.** Vorrichtung nach einem der Ansprüche 14 oder 15,
wobei die erste Einrichtung (30B) zum Erzeugen des ersten magnetischen Behandlungsfeldes einen im Wesentlichen flachen Querschnitt aufweist und die Spulen des ersten und zweiten Spulensystems in derselben Ebene angeordnet sind, welche Ebene die Spulenebene (7-7) bildet, wobei die Spulenebene quer zu der Lehnenebene angeordnet ist.

**17.** Vorrichtung nach einem der Ansprüche 14-16,
wobei das erste Spulensystem eine Basisspule (51B) und das zweite Spulensystem zwei RF-Spulen (52B, 53B) umfasst.

**18.** Vorrichtung nach Anspruch 17,
wobei die beiden RF-Spulen (52B, 53B) nebeneinander angeordnet und gegengerichtet geschaltet sind.

**19.** Vorrichtung nach Anspruch 17,
wobei die beiden RF-Spulen (52B, 53B) parallel innerhalb der Spulenöffnung der Basisspule (51B) angeordnet sind.

**20.** Vorrichtung nach einem der Ansprüche 17-19,
wobei die beiden RF-Spulen (52B, 53B) im Betrieb ein magnetisches Wechselfeld erzeugen.

**21.** Vorrichtung nach einem der Ansprüche 17-20,
wobei die erste Einrichtung (30B) zum Erzeugen des ersten magnetischen Behandlungsfeldes eine Anordnung zum Erzeugen einer Kernspinresonanz bildet, wobei im Betrieb die Basisspule (51B) ein Basismagnetfeld (B0, 55B) erzeugt, in welchem die anzuregenden Kerne präzedieren und mittels der RF-Spulen (52B, 53B) ein resonantes elektro-magnetisches Wechselfeld (B1, 56B) eingestrahlt wird.

**22.** Vorrichtung nach Anspruch 21,
wobei die magnetische Induktion des Basismagnetfeldes (B0) zwischen 0,1 Gauss und 1000 Gauss, insbesondere zwischen 1 Gauss und 100 Gauss beträgt.

**23.** Vorrichtung nach einem der vorstehenden Ansprüche,
wobei Mittel zum periodischen Erzeugen von Kernspinresonanzen umfasst sind.

**24.** Vorrichtung nach Anspruch 23,
wobei die Wiederholfrequenz der periodischen Kernspinresonanzanregung 1 Hz bis 1000 Hz, insbesondere 5 Hz bis 40 Hz beträgt.

**25.** Verfahren zur Herstellung einer Vorrichtung gemäß einem der Ansprüche 1 bis 24.

**Claims**

**1.** Apparatus for therapeutic treatment of a patient (2) using magnetic fields (B0, B1, 55B, 56B) comprising:

a first device (30B) for producing a first magnetic treatment field (B0, B1, 55B, 56B) within a first treatment region (50B),
a second device (30C) for producing a second magnetic treatment field (B0, B1, 55B, 56B) within a second treatment region (50C),
a backrest (1C) for the patient (2) to rest against in such a way that a body region of the patient (2) which is to be treated is positioned in the first treatment region (50B) and in the second treatment region (50C) when the patient (2) is in place on the apparatus and the apparatus is in an operating position,
a first arm (4B) and a second arm (4C) which protrude out of the plane defined by the backrest (1C), wherein the first device (30B) for producing the first magnetic treatment field is disposed on the first arm (4B), wherein the second device (30C) for producing the second magnetic treatment field is disposed on the second arm (4C) in such a way that a body region of the patient (2) can be positioned between the first and second devices (30B, 30C);
wherein the apparatus has a displacement device (3A, 4A), on which the first and second arms (4B, 4C) are suspended, in order to displace the first and second devices (30B, 30C) substantially along the body axis (1) of the patient (2), wherein the displacement device (3A, 4A) has at least one rail (9A, 9B) which is attached to the rear side (1G) of the backrest (1C) and the first and second arms (4B, 4C) are attached to a carriage (8A, 8B) which is disposed on the at least one rail (9A, 9B) so as to be displaceable along the backrest (1C).

2. Apparatus as claimed in claim 1, wherein the apparatus is formed as a treatment chair (1) and the backrest (1C) is formed by the backrest of the treatment chair (1).

3. Apparatus as claimed in any one of the preceding claims, wherein the arms each have a contact portion (45B, 45C) remote from the backrest and on which the first and second devices for producing the first and second magnetic treatment fields are disposed, and wherein the contact portions are moveably suspended and can be laid against the body region of the patent which is to be treated.

4. Apparatus as claimed in any one of the preceding claims, wherein the arms are pivotably attached to the apparatus.

5. Apparatus as claimed in any one of the preceding claims, wherein the arms are formed so as to be able to pivot in a plane transverse to the backrest plane and can be laid laterally against the patient (2).

6. Apparatus as claimed in any one of the preceding claims, wherein the arms have a plurality of joints (44B) which form a respective articulation chain.

7. Apparatus as claimed in claim 6, wherein a stabilising strip is woven through each of the articulation chains.

8. Apparatus as claimed in any one of the preceding claims, wherein the arms each have an outer shell, and the first and second devices for producing the first and second magnetic treatment fields and the respective joints are disposed within the respective shell.

9. Apparatus as claimed in any one of the preceding claims, wherein the arms have a substantially flat cross-section and, by pivoting, can each be laid with their flat side against the body region of the patient (2) which is to be treated.

10. Apparatus as claimed in any one of the preceding claims, wherein the apparatus has a third device (30A) for producing a third magnetic treatment field in a third treatment region (50A), wherein the third device (30A) is disposed on the backrest (1C) and wherein the first, second and third devices (30B, 30C, 30A) are disposed essentially in a U shape.

11. Apparatus as claimed in any one of the preceding claims, wherein the first and second devices (30B, 30C) are disposed laterally on the head (2A) of the patient (2) and the third device (30A) is disposed in the region of the back of the head or the spinal column of the patient (2) when the patient is in place on the apparatus and the apparatus is in the operating position.

12. Apparatus as claimed in any one of the preceding claims, wherein a locking device (7, 11, 12) is included to stop the displacement of the carriage (8A, 8B).

13. Apparatus as claimed in any one of the preceding claims, wherein the first and second arms (4B, 4C) are releasably attached to the displacement device (3A, 4A) so that the arms (4B, 4C) can be exchanged.

14. Apparatus as claimed in any one of the preceding claims, wherein the first device (30B) for producing the first magnetic treatment field has at least one first (51B) and second (52B, 53B) magnetic field generators, wherein the first and second magnetic field generators are formed as first and second coil systems.

15. Apparatus as claimed in claim 14, wherein the first magnetic treatment field is formed by superimposing the magnetic fields (B0, B1, 55B, 56B) of the first and second magnetic field generators (51B, 52B, 53B), wherein these two magnetic fields are superimposed in a substantially perpendicular manner in the first treatment region (50B).

16. Apparatus as claimed in any one of claims 14 or 15, wherein the first device (30B) for producing the first magnetic treatment field has a substantially flat cross-section and the coils of the first and second coil systems are disposed in the same plane, which plane forms the coil plane (7-7), wherein the coil plane is disposed transverse to the backrest plane.

17. Apparatus as claimed in any one of claims 14-16, wherein the first coil system has a base coil (51B) and the second coil system has two RF coils (52B, 53B).

18. Apparatus as claimed in claim 17, wherein the two RF coils (52B, 53B) are disposed next to each other and are connected in opposite directions.

19. Apparatus as claimed in claim 17, wherein the two RF coils (52B, 53B) are disposed in parallel within the coil aperture of the base coil (51B).

20. Apparatus as claimed in any one of claims 17-19, wherein the two RF coils (52B, 53B) produce an alternating magnetic field during operation.

21. Apparatus as claimed in any one of claims 17-20, wherein the first device (30B) for producing the first magnetic treatment field forms an arrangement for producing a nuclear spin resonance, wherein during operation the base coil (51B) produces a base magnetic field (B0, 55B) in which the cores to be excited precess and by means of the RF coils (52B, 53B) a resonant alternating electromagnetic field (B1, 56B) is radiated.

22. Apparatus as claimed in claim 21, wherein the magnetic induction of the base magnetic field (B0) is between 0.1 Gauss and 1000 Gauss, in particular between 1 Gauss and 100 Gauss.

23. Apparatus as claimed in any one of the preceding claims, wherein means are included for periodic production of nuclear spin resonances.

24. Apparatus as claimed in claim 23, wherein the repetition frequency of the periodic nuclear spin resonance excitation is 1 Hz to 1000 Hz, in particular 5 Hz to 40 Hz.

25. Method of producing an apparatus as claimed in any one of claims 1 to 24.

## Revendications

1. Appareil de traitement thérapeutique d'un patient (2) à l'aide de champs magnétiques (B0, B1, 55B, 56B), comprenant :

   un premier dispositif (30B) de production d'un premier champ magnétique de traitement (B0, B1, 55B, 56B) dans une première zone de traitement (50B) ;
   un deuxième dispositif (30C) de production d'un deuxième champ magnétique de traitement (B0, B1, 55B, 56B) dans une deuxième zone de traitement (50C) ;
   un dossier (1C) pour l'appui du patient (2), de manière à ce qu'une région du corps à traiter du patient (2) soit positionnée dans la première zone de traitement (50B) et dans la deuxième zone de traitement (50C) lorsque le patient (2) a pris place sur l'appareil et que l'appareil se trouve dans une position de fonctionnement,
   un premier bras (4B) et un deuxième bras (4C), faisant saillie par rapport au plan défini par le dossier (1C), le premier dispositif (30B) de production du premier champ magnétique de traitement étant disposé sur le premier bras (4B) et le deuxième dispositif (30C) de production du deuxième champ magnétique de traitement étant disposé sur le deuxième bras (4C), si bien qu'une région du corps du patient (2) peut être positionnée entre les premier et deuxième dispositifs (30B, 30C) ;

l'appareil comprenant un dispositif de translation (3A, 4A) sur lequel sont fixés les premier et deuxième bras (4B, 4C) afin de translater les premier et deuxième dispositifs (30B, 30C) sensiblement le long de l'axe du corps (1) du patient (2), le dispositif de translation (3A, 4A) comprenant au moins un rail (9A, 9B) qui est fixé sur la face arrière (1G) du dossier (1C) et les premier et deuxième bras (4B, 4C) étant fixés sur un chariot (8A, 8B) qui est monté sur ledit au moins un rail (9A, 9B) de manière à pouvoir se déplacer le long du dossier (1C).

2. Appareil selon la revendication 1, dans lequel l'appareil est conçu sous la forme d'un fauteuil de traitement (1) et le dossier (1C) est formé par le dossier du fauteuil de traitement (1).

3. Appareil selon l'une des revendications précédentes, dans lequel les bras comprennent respectivement une section d'appui (45B, 45C), éloignée du dossier, sur laquelle sont placés les premier et deuxième dispositifs de production des premier et deuxième champs magnétiques de traitement et dans lequel les sections d'appui sont accrochées de manière à être mobiles et peuvent être amenées sur la région du corps à traiter du patient.

4. Appareil selon l'une des revendications précédentes, dans lequel les bras sont montés pivotants sur l'appareil.

5. Appareil selon l'une des revendications précédentes, dans lequel les bras sont conçus pour pivoter dans un plan perpendiculaire au plan du dossier et peuvent être amenés latéralement contre le patient (2).

6. Appareil selon l'une des revendications précédentes, dans lequel les bras comprennent plusieurs articulations (44B) et forment chacun une chaîne d'articulations.

7. Appareil selon la revendication 6, dans lequel un feuillard de stabilisation est chaque fois entrelacé dans les chaînes d'articulation.

8. Appareil selon l'une des revendications précédentes, dans lequel les bras comprennent chacun une coque extérieure et dans lequel les premier et deuxième dispositifs de production des premier et deuxième champs magnétiques de traitement ainsi que les articulations respectives sont logés chacun à l'intérieur d'une coque.

9. Appareil selon l'une des revendications précédentes, dans lequel les bras possèdent une section transversale sensiblement plate et peuvent grâce au pivotement être amenés par leur côté plat sur la région du corps à traiter du patient (2).

10. Appareil selon l'une des revendications précédentes, dans lequel l'appareil comprend un troisième dispositif (30A) de production d'un troisième champ magnétique de traitement dans une troisième zone de traitement (50A), le troisième dispositif (30A) étant disposé sur le dossier (1C) et les premier, deuxième et troisième dispositifs (30B, 30C, 30A) étant sensiblement disposés en forme de U.

11. Appareil selon l'une des revendications précédentes, dans lequel les premier et deuxième dispositifs (30B, 30C) sont disposés latéralement au niveau de la tête (20A) du patient (2) et le troisième dispositif (30A) au niveau de l'occiput ou de la colonne vertébrale du patient (2), lorsque le patient a pris place sur l'appareil et que l'appareil se trouve dans une position de fonctionnement.

12. Appareil selon l'une des revendications précédentes, dans lequel il est prévu un dispositif de verrouillage (7, 11, 12) afin de bloquer la translation du chariot (8A, 8B).

13. Appareil selon l'une des revendications précédentes, dans lequel les premier et deuxième bras (4B, 4C) sont fixés de manière amovible sur le dispositif de translation (3A, 4A) afin de permettre un remplacement des bras (4B, 4C).

14. Appareil selon l'une des revendications précédentes, dans lequel le premier dispositif (30B) de production du premier champ magnétique de traitement comprend au moins un premier (51B) et un deuxième (52B, 53B) générateurs de champ magnétique, les premier et deuxième générateurs de champ magnétique prenant la forme d'un premier et d'un deuxième système de bobines.

15. Appareil selon la revendication 14, dans lequel le premier champ magnétique de traitement est formé par une superposition des champs magnétiques (B0, B1, 55B, 56B) des premier et deuxième générateurs de champ magnétique (51B, 52B, 53B), ces deux champs magnétiques se superposant sensiblement perpendiculairement dans la première zone de traitement (50B).

16. Appareil selon la revendication 14 ou 15, dans lequel le premier dispositif (30B) de production du premier champ magnétique de traitement présente une section transversale sensiblement plate et les bobines des premier et deuxième systèmes de bobines sont disposés dans un même plan, ce plan constituant le plan des bobines (7-7) et le plan des bobines étant perpendiculaire au plan du dossier.

17. Appareil selon l'une des revendications 14 à 16, dans lequel le premier système de bobines comprend une bobine de base (51B) et le deuxième système de bobines comprend deux bobines RF (52B, 53B).

18. Appareil selon la revendication 17, dans lequel les deux bobines RF (52B, 53B) sont juxtaposées et sont montées en opposition.

19. Appareil selon la revendication 17, dans lequel les deux bobines RF (52B, 53B) sont montées parallèles à l'intérieur de l'ouverture de la bobine de base (51B).

20. Appareil selon l'une des revendications 17 à 19, dans lequel les deux bobines RF (52B, 53B) produisent en service un champ magnétique alternatif.

21. Appareil selon l'une des revendications 17 à 20, dans lequel le premier dispositif (30B) de production du premier champ magnétique de traitement forme un ensemble de production d'une résonance magnétique nucléaire, dans lequel la bobine de base (51B) produit en service un champ magnétique de base (B0, 55B), où les noyaux à exciter sont en précession, et un champ électro-magnétique alternatif (B1, 56B) résonant est produit à l'aide des bobines RF (52B, 53B).

22. Appareil selon la revendication 21, dans lequel l'induction magnétique du champ magnétique de base (B0) se situe entre 0,1 gauss et 1000 gauss, en particulier entre 1 gauss et 100 gauss.

23. Appareil selon l'une des revendications précédentes, dans lequel il est prévu des moyens de production périodique de résonances magnétiques nucléaires.

24. Appareil selon la revendication 23, dans lequel la fréquence de répétition de l'excitation périodique de la résonance magnétique nucléaire est de 1 Hz à 1000 Hz, en particulier de 5 Hz à 40 Hz.

25. Procédé de fabrication d'un appareil selon l'une des revendications 1 à 24.

Fig. 1

Fig. 2

Fig. 3

Fig. 4

Fig. 5

Fig. 6

Fig. 7

Fig. 8

Fig. 9

360

260

170

560

57 B

Fig. 10

Fig. 11

# Fig. 12

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4026173
- WO 9966986 A
- WO 02096514 A
- GB 429044 A

**In der Beschreibung aufgeführte Nicht-Patentliteratur**

- *Orthopädische Praxis,* August 2000, vol. 36, 510-515
- **FRITZ LECHNER.** *Elektrostimulation und Magnetfeldtherapie. Anwendung, Ergebnisse und Qualitätssicherung,* 1989